# EUROPÄISCHE PATENTSCHRIFT

(11) **EP 1 960 521 B1**
(45) Veröffentlichungstag und Bekanntmachung des Hinweises auf die Patenterteilung: **21.09.2011**
(21) Anmeldenummer: 06830455.9
(22) Anmeldetag: 07.12.2006
(51) Int. Cl.: C12N 15/10

(54) **VERFAHREN UND TESTKIT ZUR TRENNUNG, AUFREINIGUNG UND WIEDERGEWINNUNG VON LANG- UND KURZKETTIGEN NUKLEINSÄUREN**
METHOD AND TEST KIT FOR THE SEPARATION, PURIFICATION AND RECOVERY OF LONG- AND SHORT-CHAIN NUCLEIC ACIDS
PROCEDE ET KIT D'ANALYSE POUR LA SEPARATION, LA PURIFICATION ET LA RECUPERATION D'ACIDES NUCLEIQUES A CHAINE LONGUE ET A CHAINE COURTE

(30) Priorität: 07.12.2005 DE 102005059217
(43) Veröffentlichungstag der Anmeldung: 27.08.2008
(73) Patentinhaber: AJ Innuscreen GmbH, 13125 Berlin (DE)
(72) Erfinder: HILLEBRAND, Timo, D-15366 Hoenow (DE)
(74) Vertreter: Wehlan, Helmut
(86) Internationale Anmeldenummer: PCT/EP2006/069451
(87) Internationale Veröffentlichungsnummer: WO 2007/065934

(56) Entgegenhaltungen:
- WO-A-00/34463
- WO-A-02/04620
- WO-A-89/08257
- WO-A-2004/042058
- WO-A-2004/055207
- WO-A1-99/54340
- WO-A1-2005/058933
- WO-A1-2005/058933
- DE-A1- 19 746 874
- DE-A1- 19 746 874
- EON-DUVAL ALEX ET AL: "Precipitation of RNA impurities with high salt in a plasmid DNA purification process: Use of experimental design to determine reaction conditions." BIOTECHNOLOGY AND BIOENGINEERING, Bd. 83, Nr. 5, 5. September 2003 (2003-09-05), Seiten 544-553, XP002422884 ISSN: 0006-3592
- FARRAH S R: "Chemical factors influencing adsorption of bacteriophage MS2 to membrane filters." APPLIED AND ENVIRONMENTAL MICROBIOLOGY MAR 1982, Bd. 43, Nr. 3, März 1982 (1982-03), Seiten 659-663, XP002422885 ISSN: 0099-2240
- LAKSHMI S ET AL: "STUDIES ON THE CHAOTROPICALLY SOLUBILIZED ARYL SULFATASE C EC-3.1.6.1 AND ESTRONE SULFATASE OF SHEEP BRAIN" BIOCHIMICA ET BIOPHYSICA ACTA, Bd. 567, Nr. 1, 1979, Seiten 184-195, XP002422886 ISSN: 0006-3002

## Beschreibung

Die Erfindung betrifft eine neuartige Pufferformulierung zur schnellen Trennung, Aufreinigung und hocheffizienten Rückgewinnung von lang- und / oder kurzkettigen Nukleinsäuren.

Die Erfindung zielt insbesondere auf solche Anwendungen, bei denen spezifische Nukleinsäurefragmente aus komplexen Reaktionsansätzen (PCR, Restriktionsansätze, Sequenzierungsansätze, Markierungsansätze) hocheffizient und schnell aufgereinigt und wiedergewonnen werden sollen, um sie einer nachfolgenden Reaktion zuzuführen.

Für die Aufreinigung und Rückgewinnung von spezifischen DNA-Fragmenten existieren heute eine Vielzahl von kommerziell verfügbarer Kits.

Alle diese Verfahren basieren auf einer von Vogelstein und Gillespie (Proc. Natl. Acad. Sci. USA, 1979, 76, 615-619) entwickelten und erstmals beschriebenen Methode zur präparativen und analytischen Reinigung von DNA-Fragmenten aus Agarosegelen. Die Methode kombiniert die Auflösung der die zu isolierende DNA- Bande enthaltende Agarose in einer gesättigten Lösung eines chaotropen Salzes (NaJ) mit einer Bindung der DNA an Glaspartikel. Die an die Glaspartikel fixierte DNA wird anschließend mit einer Waschlösung (20 mM Tris HCl [pH 7,2]; 200mM NaCl; 2 mM EDTA; 50% v/v Ethanol) gewaschen und abschließend von den Trägerpartikeln abgelöst.

Das physiko-chemische Prinzip der nach dem bekannten Stand der Technik heute eingesetzten und kommerziell verfügbaren Systeme zur Isolierung von Nukleinsäuren auf der Basis der Bindung von Nukleinsäuren an die Oberflächen mineralischer Träger soll dabei in der Störung übergeordneter Strukturen des wässerigen Milieus bestehen, durch welche die Nukleinsäuren an der Oberfläche von mineralischen Materialien, insbesondere von Glas- bzw. Silicapartikeln adsorbieren. Die Störung der übergeordneten Strukturen des wässerigen Milieus erfolgt dabei immer unter Anwesenheit chaotroper Ionen und ist bei hohen Konzentrationen dieser fast quantitativ. Auf dieser beschriebenen physiko-chemischen Basis enthalten alle kommerziell verfügbaren Systeme zur Isolierung von Nukleinsäuren Pufferkompositionen mit hohen Ionenstärken chaotroper Salze, für die Bindung von Nukleinsäuren an eine Nukleinsäuren-bindende feste Phase.

All den beschriebenen Verfahren zur Isolierung von Nukleinsäuren über die Bindung der Nukleinsäuren an mineralische feste Phase unter Verwendung chaotroper Salzlösungen ist gemeinsam, dass für die Bindung der Nukleinsäuren an die verwendeten Trägermaterialien hohe Konzentrationen eingesetzt werden müssen. Dabei sind gerade chaotrope Salze (z.B. Guanidinisothiocyanat, Guandinhydrochlorid, Natriumperchlorat oder Natriumjodid) hochtoxisch wirksame Substanzen. Die Verwendung findenden Puffersysteme mit sehr hohen Ionenstärken bewirken oftmals ein Verschleppen von Salzkontaminationen, was sich problematisch für eine Reihe von down-stream-Applikationen erweisen kann. Darüber hinaus besteht beim Umgang mit chaotropen Puffern ein erhebliches gesundheitliches Risiko (insbesondere bei Langzeitanwendungen ) sowie eine erhebliche Umweltbelastung durch in Abwasser eingebrachte Schadstofflasten.

Interessanterweise zeigt sich, dass alle weltweit kommerziell verfügbaren Systeme zur Isolierung von Nukleinsäuren basierend auf der Bindung der Nukleinsäuren an mineralische Trägermaterialien (Magnetpartikel, Membranen, Carrier-Suspensionen u.a.) prinzipiell nach dem beschriebenen Verfahren arbeiten. Seit der Erstbeschreibung durch Vogelstein und Gillespie werden die gebundenen Nukleinsäuren immer mit Alkohol oder acetonhaltigen Salzlösungen gewaschen. Die Waschschritte sind essentieller Bestandteil der Extraktionsprotokolle und dienen neben der Entfernung von gebundenen unerwünschten inhibitorischen Stoffen immer auch der notwendigen Entfernung der für die Bindung der Nukleinsäuren notwendigen Salze.

In der Patentschrift WO 01/62976 A1 wird ein Verfahren offenbart, welches die Aufreinigung von Nukleinsäuren aus unterschiedlichen Reaktionsansätzen unter Zugabe von unterschiedlichen Alkoholen, deren nachfolgender Präzipitation an speziellen festen Phasen (Membranen mit spezifischen physikalischen Charakteristika), Waschschritten mit alkoholischen Puffern und der fmalen Elution der Nukleinsäuren mittels Wasser, umfasst.

Ebenso beschreiben die Patenschriften US 5405951 A und EP 0512767 A1 die Isolierung von Nukleinsäuren durch Inkubation der Nukleinsäure enthaltenden Probe mit einem Alkohol und der nachfolgenden Inkubation der Probe mit einem mineralischen Material. Die Elution der Nukleinsäuren erfolgt über die Zugabe von auf 60°C erwärmtem Wasser.

In der Patentschrift DE 10253351 A1 wird offenbart, dass die Aufreinigung und Rückgewinnung von Nukleinsäuren dadurch erfolgt, dass man die Nukleinsäure enthaltende Lösung mit Zusätzen so einstellt, dass sie monovalente und multivalente Kationen sowie einen Alkohol enthält, sie danach mit der festen Phase in Kontakt bringt, den Träger anschließend ggf. wäscht und die Nukleinsäure von der festen Phase löst. Als monovalente Salzkomponenten werden Ammoniumchlorid, Natriumchlorid und/oder Kaliumchlorid verwendet und als multivalente Salzkomponente Magnesiumchlorid, Calciumchlorid, Zinkchlorid und/oder Manganchlorid.

Es wird offenbart, dass gerade die Kombination eines monovalenten und eines multivalenten Salzes dazu führt, dass Nukleinsäuren an feste Phasen adsorbieren, wobei die dazu notwendigen Ionenstärken nur sehr gering sein müssen. Dies hat den Vorteil, dass ggf. bisher immer notwendige Waschschritte nicht mehr benötigt werden und sich somit die Verfahren zur Isolierung von Nukleinsäuren deutlich verkürzen und vereinfachen lassen.

Allerdings zeigt sich, dass bei der Verwendung von in der Patenschrift DE 10253351 A1 angegebenen Pufferkombinationen (z.B. Magnesiumchlorid/Kaliumchlorid) die Aufreinigung und Rückgewinnung von DNA-Fragmenten aus PCR-Reaktionsgemischen zwar mit einer hohen Rückgewinnungsrate erfolgt, aber leider keine selektive Entfernung von unerwünschten PCR-Nebenprodukten (z.B. Primer-Dimere) möglich ist.

Damit kann nur die generelle Möglichkeit der Rückgewinnung von DNA-Fragmenten, nicht aber deren effiziente Aufreinigung nachgewiesen werden. Ursache für diese Beobachtung scheint dabei möglicherweise das verwendete multivalente Kation zu sein. Wird allerdings das multivalente Kation aus dem Puffergemisch entfernt, dann ist die Rückgewinnung von Nukleinsäuren mit den beschriebenen Puffern auf der Basis der sehr geringen Ionenstärken nicht mehr möglich. Gerade aber die Verwendung von Puffern mit nur sehr geringen Ionenstärken war die erfindungsgemäße Bestimmung der Patentschrift.

Die Druckschrift WO00/34463 A1 beschreibt ein Verfahren zur Isolierung von Nukleinsäuren, bei dem die Nukleinsäuren an eine feste Phase gebunden werden, wobei die Vermittlung der Bindung durch Bindungspuffer auf der Basis sogenannter antichaotroper Salze und einer alkoholischen Komponente erfolgt. Die gebundenen Nukleinsäuren werden mit an sich bekannten Waschpuffern gewaschen und final durch die Zugabe eines Niedrigsalzpuffers eluiert. Die Ionenstärken der sogenannten antichaotropen Salze betragen mindestens 0.1 M - 10 M. Die sogenannten antichaotropen Salze für die Bindung von Nukleinsäuren an eine feste Phase sind ausnahmslos Chloride.

Die Patentschrift WO 89/08257 A1 verweist darauf, dass Citrate unter die Rubrik antichaotrope Salze fallen. Die in dieser Schrift charakterisierte Eigenschaft versteht sich im Kontext der Immobilisierung von Proteinen und hat keinerlei Bezug auf Nukleinsäuren oder Verfahren zur Isolierung und Aufreinigung von Nukleinsäuren. <Seiten 4a und 4b einfügen>

Der vorliegenden Erfindung lag die Aufgabe zu Grunde, die o.g. Nachteile des Stands der Technik zu beseitigen.

Die Aufgabe wurde durch die Merkmale der Patentansprüche gelöst. Erfindungsgemäß wurde ein Verfahren und ein Testkit bereitgestellt, dass sowohl eine hocheffiziente Rückgewinnung von lang- oder kurzkettigen Nukleinsäuren ermöglicht, dabei gegebenenfalls selektiv unerwünschte Nukleinsäuren abtrennt und einfach und schnell in der Durchführung ist.

Das erfindungsgemäße Verfahren zur Trennung, Aufreinigung und Wiedergewinnung von lang- und / oder kurzkettigen Nukleinsäuren besteht aus folgenden Schritten:
(1) Bindung der Nukleinsäuren an eine feste Phase mittels eines Bindungspuffers
(2) Elution der gebundenen Nukleinsäuren von der festen Phase,
wobei der Bindungspuffer
a) als wirksame Komponente eine Zusammensetzung aus mindestens einem Salz der Zitronensäure mit einfach positiv geladenen Kationen und
b) mindestens einem Alkohol umfasst und
c) weder chaotrope Salze noch eine Kombination von Salzen mit mono- und multivalenten Kationen enthält und
d) dass die Ionenstärken für die Anbindung an die feste Phase in Kombination mit einem Alkohol kleiner als 100mM sind.

Erfindungsgemäß können folgende Salze eingesetzt werden:

| | |
|---|---|
| a) Di-Ammoniumhydrogencitrat | und / oder |
| b) Ammoniumdihydrogencitrat | und / oder |
| c) Tri-Natriumcitrat | und / oder |
| d) Di-Natriumhydrogencitrat | und / oder |
| e) Natriumdihydrogencitrat | und / oder |
| f) Tri-Kaliumcitrat | und / oder |
| g) Di-Kaliumhydrogencitrat | und / oder |
| h) Kaliumdihydrogencitrat. | |

Die Druckschrift W02005058933 A offenbart eine Methode zur Isolierung von RNA aus einer biologischen Probe durch folgende Schritte: (a) in Kontaktbringen eines Gemisches zur Denaturierung von Proteinen, bestehend aus einem Lithiumsalz (2.5-4.0 M), einem Alkohol (25-40% v/v), und einem Zitratsalz (25-100 mM) mit einer festen Phase, so dass Proteine auf der festen Phase denaturiert werden; (b) in Kontaktbringen der festen Phase mit einer aus RNA bestehenden Probe, so dass die RNA an die feste Phase bindet; (c) waschen der festen Phase um biologisches Material, das keine RNA darstellt, zu entfernen; und (d) eluieren der gebundenen RNA von der festen Phase. Die Ionenstärke in W02005058933 A zur Bindung der Nukleinsäure an die feste Phase liegt durch die hohe Konzentration an Litiumchlorid mindestens eine Zehnerpotenz höher als 100 mM.

Die Offenlegungsschrift DE 19746874 A1 beschreibt die Reinigung von Nukleinsäuren an hydrophoben Membranen. Die beschriebenen Verfahren nutzen Gemische von Salzen und Alkoholen zur Anbindung von Nukleinsäuren an die hydrophoben Membranen. Unter den beschriebenen Bedingungen binden die Nukleinsäuren an die hydrophobe Membran, werden gewaschen und dann von der Oberfläche der Membran abpipettiert. Es gibt keinerlei Hinweis auf die Verwendung von Salzen der Zitronensäure in Kombination mit einem Alkohol zur Anbindung der Nukleinsäuren an eine feste Phase. Es kommen die bereits bekannten Kombination an Salzen/Alkoholen zum Einsatz (z.B. hochmolare Lösungen an Guanidiniumsalzen in Kombination mit einem Alkohol). Die Citrat-Konzentrationen liegen in einem Bereich von 0,8 M. Alle dort aufgeführten Puffer enthalten Salze in hohen Ionenstärken.

Die Publikation von Farrah, S. R. in Applied and environmental microbiology, Mar. 1982, Bd. 43, Nr. 3, März 1982 (1982-03), Seiten 659-663, erwähnt, dass antichaotrope Salze, wie MgSO₄ und Metall-Chelatbildner wie Citrat-Ionen die Adsorption der Bakteriophage MS2 an Membran-Filter unterstützen.

S. Lakshmi and A.S. Balasubramanian beschreiben in Biochimica et Biophysica Acta, Bd. 567, Nr. 1, 1979, Seiten 184-195, dass Citrate die Aktivität des gelösten Enzyms Arylsulfatase signifikant steigern.

Die internationale Patentanmeldung WO 2004/042058 A offenbart ein Verfahren zur Isolierung von Nukleinsäuren aus einer Lösung durch Bindung an eine feste Phase mittels nichtchaotropen Salzen, dadurch gekennzeichnet, dass man die Nukleinsäure enthaltende Lösung mit Zusätzen so einstellt, dass sie monovalente und multivalente Kationen sowie einen Alkohol und ggf. weitere Zusatze enthält, sie danach mit der festen Phase in Kontakt bringt, den Träger anschließend ggf. wäscht und die Nukleinsaure von der festen Phase löst oder dass sie multivalente und/oder monovalente Kationen, ggf. einen Alkohol und ggf. weitere Zusätze enthalt und ein bestimmter pH-Wert zwischen 5 und 10 eingestellt wird. Die Verwendung von Citraten wird nicht offenbart.

Die Druckschrift WO 02/04620 A beschreibt ein Verfahren zur Isolierung von Nukleinsäuren aus einer Lösung durch Bindung an eine feste Phase mittels nichtchaotroper Salze, das sich durch folgende Schritte kennzeichnet: a) Adsorbieren der in der Lösung enthaltenen Nukleinsäuren in Gegenwart von Alkali- und/oder Erdalkalisalzen an einer SiO₂-haltigen Oberfläche, und b) Eluieren der Nukleinsäuren mit einer wässrigen Lösung und gegebenenfalls Isolieren der Nukleinsäuren, dadurch gekennzeichnet, dass in Schritt a) die Adsorption der Nukleinsäuren an die SiO₂-haltige Oberfläche in Gegenwart von 0,1 bis 3 M nichtchaotropen Alkali- und/oder Erdalkalisalzen und 37 bis 70 Vol-% eines aliphatischen Alkohols erfolgt. Die Verwendung von Citraten wird nicht offenbart.

Zum Stand der Technik gehört auch die Veröffentlichung von Eon-Ouval Alex et al: in Biotechnology and Bioengineering, Bd. 83, Nr. 5, 5. September 2003, Seiten 544-553. Darin wird eine Hochsalz-Lösung zur Abtrennung von RNA bei einem pharmazeutischen Reinigungsprozess von Plasmid-DNA beschrieben. Auch eine Lösung von Kaliumcitrat in einer Konzentration von 1,5M wird dabei beschrieben. Alle dort aufgeführten Lösungen enthalten Salze in hohen Ionenstärken.

Die Alkoholkonzentrationen der Bindungspuffer liegt zwischen 20% - 90%, vorzugsweise zwischen 40% - 70%. Als Alkohole können Methanol, Ethanol, Propanol, Isopropanol, Ethylenglycol, Polyethylenglycol oder Gycerin verwendet werden.

Erfindungsgemäß betragen die Ionenstärken für die Anbindung an die feste Phase in Kombination mit einem Alkohol weniger als 100mM, vorzugsweise weniger als 50 mM.

Die feste Phase kann aus Glasfasermaterialien, Silicagele, Suspensionen aus mineralischen Trägern, funktionalisierte magnetische Partikel; vorzugsweise Glasfasermaterialien von 0.7 µm bis 2 µm, bestehen.

Das erfindungsgemäße Verfahren zur Trennung, Aufreinigung und Wiedergewinnung von lang- und / oder kurzkettigen Nukleinsäuren ist durch folgende Schritte gekennzeichnet:
➢ Zugabe der Bindungspuffer gemäß einem der Ansprüche 1 bis 9 zu Reaktionsansätzen, die Nukleinsäuren enthalten
➢ Überführung der Mischung aus den Reaktionsansätzen und den Bindungspuffern an eine feste Phase
➢ Elution der an die feste Phase gebundenen Nukleinsäuren.

Ein Waschen der an die feste Phase gebundenen Nukleinsäuren entfällt.

Gegenstand der Erfindung ist auch ein Testkit, umfassend mindestens einen Alkohol, mindestens ein Salz der Zitronensäure, eine feste Phase und bekannte Elutionspuffer.

Die erfindungsgemäße Verwendung von Salzen der Zitronensäure in Kombination mit mindestens einem Alkohol ist die Trennung, Aufreinigung und Wiedergewinnung von lang-und / oder kurzkettigen Nukleinsäuren, insbesondere von PCR-Produkten, Restriktionsansätzen oder Sequenzieransätzen.

Überaschenderweise zeigte sich, dass verschiedene Salze der Zitronensäure in Kombination mit einem Alkohol in der Lage sind, lang - und kurzkettige Nukleinsäuren an bekannte Trägermaterialien, insbesondere Glasfasermaterialen zu binden und wieder abzulösen.

Überraschenderweise zeigte sich auch, dass die für die Anbindung der Nukleinsäuren benötigten Ionenstärken ebenfalls nur in millimolaren Konzentrationen vorliegen müssen. Die Verwendung von Pufferlösungen auf der Basis eines Alkohols und eines Salzes der Zitronensäure ermöglicht eine hocheffiziente Rückgewinnung von Nukleinsäuren aus Reaktionsansätzen bei gleichzeitiger effizienter Abtrennung von unerwünschten Nebenprodukten. Dies ist insbesondere bei der Aufreinigung von PCR-Produkten aus PCR-Reaktionsgemischen der Fall, wo insbesondere Primer oder Primerdimere von den spezifischen Amplifikationsprodukten getrennt werden sollen.

Als bevorzugte Salze der Zitronensäure werden z.B. Di-Ammoniumhydrogencitrat, Tri-Kaliumcitrat-Monohydrat oder Tri-Natriumcitrat-Dihydrat eingesetzt. Auch sind Ammoniumdihydrogencitrat, Di-Natriumydrogencitrat, Natriumdihydrogencitrat, Di-Kaliumydrogencitrat, Kaliumdihydrogencitrat einsetzbar. Die für die Anbindung notwendigen Ionenstärken sind kleiner 100 mM, vorzugsweise kleiner 50 mM. Die benötigten Alkoholkonzentrationen der Bindungspuffer liegen zwischen 20% - 90%, vorzugsweise zwischen 40% -70%. Es können unterschiedliche Alkohole eingesetzt werden, vorzugsweise wird Isopropanol verwendet.

Das erfindungsgemäße Verfahren zur Aufreinigung und Rückgewinnung von DNA-Fragmenten aus Reaktionsansätzen ist extrem schnell und einfach in der Durchführung. Die Reaktionsansätze, aus welchen die Nukleinsäure(en) aufgereinigt werden sollen, werden mit dem erfindungsgemäßen Bindungspuffer gemischt und nachfolgend auf eine Zentrifugationssäule (z.B. mit einem Glasfasermaterial) überführt und zentrifugiert. Danach wird die Zentrifugationssäule in ein neues Auffanggefäß verbracht und die DNA-Fragmente nach Zugabe von Wasser oder einem Niedrigsalzpuffer (10 mM Tris.HCl) von der Säulenoberfläche eluiert.

Im Gegensatz zu den kommerziell Verwendung findenden Verfahren (Kits) sind keine Waschschritte erforderlich. Das Verfahren verzichtet darüber hinaus vollständig auf gesundheits- oder umweltgefährdende chaotrope Salze, wie sie bisher in verfügbaren kommerziellen Verfahren eingesetzt werden.

Darüber hinaus kann der zeitliche Aufwand für eine Aufreinigungsreaktion drastisch reduziert werden. Eine Aufreinigung kann i.d.R. in ca. 3 min abgeschlossen sein.

Dass Verfahren erlaubt die Aufreinigung und Rückgewinnung eines breiten Größenspektrums von DNA-Fragmenten mit einer sehr hohen Rückgewinnungsrate.

Überraschenderweise zeigte sich auch, dass die Kombination der erfindungsgemäßen Salze der Zitronensäure in Hinblick auf die Ionenstärke mit der jeweiligen Alkoholkonzentration sowohl die Effizienz der Rückgewinnung als auch die Selektivität in Bezug auf die auf zureinigenden DNA-Fragmente in Bezug auf deren Fragmentlänge beeinflusst. Diese Beobachtung kann in hervorragender Weise dafür genutzt werden, neue Anwendungsgebiete zu erschließen. So ermöglicht das erfindungsgemäße Verfahren auch eine effiziente Aufreinigung von PCR-Produkten, Restriktionsansätzen oder Sequenzieransätzen. Im Fall der Sequenzieransätze steht die Aufgabe der effizienten Abtrennung von Dye-Terminatoren bei gleichzeitiger effizienten Rückgewinnung der Nukleinsäurefragmente eines weiten Molekulargewichtsspektrums, insbesondere auch die Rückgewinnung von sehr kleinen Nukleinsäurefragmenten. Die Realisierung dieser Aufgabenstellung benötigt ebenfalls nur 3 min und ist damit deutlichst einfacher und schneller als alle anderen bisher Verwendung findenden Verfahren.

Der Unterschied zwischen der vorliegenden Erfindung und der in WO00/34463 A1 vorgeschlagenen Lösung besteht unter anderem darin, dass die Ionenstärken, kleiner als 0.1 M, vorzugsweise kleiner als 0,05 M sind. In WO00/34463 A1 ist die Verwendung von Salzen der Zitronensäure ist nicht offenbart. Dies ist auch erklärbar, da die in dieser Schrift aufgeführten Anwendungen mit Salzen der Zitronensäure überhaupt nicht durchführbar sind. Die angemeldete Erfindung bezieht sich aber nur auf Salze der Zitronensäure. WO00/34463 A1 beschreibt ausnahmslos Verfahren zur Isolierung und Aufreinigung von genomischen Nukleinsäuren, welche aus komplexen biologischen Proben isoliert werden. Es beschreibt aber kein Verfahren, welches die Aufreinigung und Wiedergewinnung einer bereits vorliegenden Nukleinsäure ermöglicht. Aus diesem Grund erfolgt die Isolierung der Nukleinsäuren auch immer mit Puffern, die neben einer Salzkomponente auch noch Detergenzien, proteolytische Enzyme sowie weitere Zusätze enthalten, deren Funktion im Aufschluss (Lyse) der biologischen Probe besteht.

Die vorliegende Erfindung bezieht nicht auf die Isolierung von genomischen Nukleinsäuren aus komplexen biologischen Proben. Der Gegenstand der vorliegenden Erfindung ist die Aufreinigung von Reaktionsansätzen, z.B. PCR-Reaktionsansätzen, wobei nachfolgend z.B. ein amplifiziertes DNA-Fragment mit einer hohen Rückgewinnungsrate wiedergewonnen werden soll. Es erfolgt keine Lyse einer biologischen Probe, wie dies mit den Puffern der aufgeführten Druckschrift WO00/34463 A1 realisiert wird. WO00/34463 A1 beschreibt ausnahmslos ein Verfahren zur Isolierung und Aufreinigung von genomischen Nukleinsäuren, welches als obligaten Schritt das Waschen (mehrmalige Waschen) der an der festen Phase gebundenen Nukleinsäuren enthält. Das Waschen ist nicht nur notwendig, um inhibitorische Stoffe aus der biologischen Proben zu entfernen, es ist auch notwendig, um die hohen Salzkonzentrationen der verwendeten Bindungspuffer auszuwaschen. Die Analyse der Ausführungsbeispiele zeigt, dass die aufgeführten Lyse/Bindungspuffer Ionenstärken von i.R. > 1.5 M aufweisen. Die vorliegende Erfindung verwendet deutlich geringere Salzkonzentrationen (kleiner 0.1 M). Aus diesem Grunde besteht der erfindungsgemäße Vorteil auch darin, ohne bisher notwendige Waschschritte auskommen zu können und damit das Verfahren deutlich zu vereinfachen und den Ablauf zu verkürzen.

Darüber hinaus gibt es generell im Stand der Technik keinerlei Hinweise darauf, dass für die Anbindung von Nukleinsäuren an an sich bekannte mineralische feste Phasen, Salze der Zitronensäure eingesetzt werden. Die Erfindung basiert aber gerade auf dieser Beobachtung, umso mehr, dass gerade diese Salze es ermöglichen, eine Anbindung von Nukleinsäuren auch bei vorliegenden Ionenstärken von kleiner 100 mM, insbesondere kleiner 50 mM, zu realisieren. Die in WO00/34463 A1 aufgeführten Salze erlauben keine Anbindung von Nukleinsäuren und deren quantitative Rückgewinnung, wenn sie in diesen niedrigen Ionenstärken eingesetzt werden würden.

Die Schrift WO 89/08257 A1 verweist darauf, dass Citrate unter die Rubrik antichaotrope Salze fallen. Die in dieser Schrift charakterisierte Eigenschaft, versteht sich im Kontext der Immobilisierung von Proteinen und hat keinerlei bezug auf Nukleinsäuren oder Verfahren zur Isolierung und Aufreinigung von Nukleinsäuren.

Auch andere Dokumente, neben der schon ausführlich beschriebenen Druckschrift WO00/34463 A1, beschreiben die Nutzung von sogenannten antichaotropen wie auch chaotropen Salzen zur Isolierung und Aufreinigung von Nukleinsäuren. Insofern ist es auch nicht das Ziel der vorliegenden Erfindung,. antichaotrope Salze und deren Verwendung für die Nukleinsäure-Isolierung zu nutzen, sondern vielmehr die Möglichkeit bereitzustellen, dass erstmals die Kombination von Salzen der Zitronensäure und einem Alkohol, in extrem geringen Ionenstärken, für eine effiziente Aufreinigung und nachfolgende quantitative Rückgewinnung von Nukleinsäuren, und dabei von Nukleinsäurefragmenten, zu nutzen. Und dies war bisher nicht bekannt.

Die Erfindung wird nachfolgend anhand eines Ausführungsbeispieles erklärt.

### Ausführungsbeispiel

Aufreinigung und Rückgewinnung eines PCR-Produktes von 98 bp aus einem PCR-Reaktionsgemisch. Verwendung unterschiedlicher Bindungspuffer.
Puffer 1: 50 mM Di-Ammoniumhydrogencitrat / 62% Isopropanol
Puffer 2: 50 mM Tri-Natriumcitrat-Dihydrat / 62% Isopropanol
Puffer 3: 50 mM Tri-Kaliumcitrat-Monohydrat / 62% Isopropanol
Puffer 4: 25 mM Di-Ammoniumhydrogencitrat / 62% Isopropanol
Puffer 5: 25 mM Tri-Natriumcitrat-Dihydrat / 62% Isopropanol
Puffer 6: 25 mM Tri-Kaliumcitrat-Monohydrat / 62% Isopropanol

Jeweils 500 µl des Bindungspuffers wurden mit jeweils 50 µl eines PCR-Ansatzes mit einem amplifizierten Fragment von 98 bp gemischt.

Die Mischung wurde nachfolgend zur Anbindung des gewünschten Nukleinsäurefragmentes auf eine Zentrifugationssäule mit einem Glasfaserfilter (AF; Fa. Pall) überführt und bei 10. 000 x g für 1 min zentrifugiert. Die Zentrifugationssäule wurde danach in ein neues 1.5 ml Reaktionsgefäß gesteckt und nach Zugabe eines Elutionsmittels (10 mM Tris-HCl) nochmals für 1 min bei 8.000 x g zentrifugiert.

Die eluierten PCR-Fragmente wurden nachfolgend auf einem Agilent Bioanalyzer ausgewertet und die Reinheit sowie die Rückgewinnungsraten in Bezug auf den unaufgereinigten PCR-Ansatz bestimmt.

Es konnten keine unspezifischen Primer und Primerdimere mehr detektiert werden.

Die nachfolgende Tabelle enthält die Rückgewinnungsraten für die jeweiligen unterschiedlichen Bindungspuffer P1 - P6.

| Ausgangsfragment; unaufgereinigt | Bindungspuffer | Bindungspuffer | Bindungspuffer | Bindungspuffer | Bindungspuffer | Bindungspuffer |
|---|---|---|---|---|---|---|
| | P1 | P2 | P3 | P4 | P5 | P6 |
| 100 % | 83,5 % | 78,5 % | 76,9 % | 87,8 % | 74,4 % | 82,6 % |

Das Beispiel illustriert deutlich, das mit den erfindungsgemäßen Bindungspuffern sehr hohe Rückgewinnungsraten zu erzielen sind.

### Definitionen

### Chaotrope Salze:

Salze, die regelmäßige - auf der Bildung von Wasserstoffbrückenbindungen beruhende - Struktur von flüssigem Wasser zerstören, indem sie die Bildung der zur Solvatation notwendigen H₂O-Käfigstrukturen verhindern. Beispiele für chaotrope Bestandteile sind Thiocyanate, Iodide oder Perchlorate.

## Patentansprüche

1. Verfahren zur Trennung, Aufreinigung und Wiedergewinnung von lang- und / oder kurzkettigen Nukleinsäuren mit folgenden Schritten:
➢ Bindung der Nukleinsäuren an eine feste Phase mittels eines Bindungspuffers
➢ Elution der gebundenen Nukleinsäuren von der festen Phase,
**dadurch gekennzeichnet, dass** der Bindungspuffer
a) als wirksame Komponente eine Zusammensetzung aus mindestens einem Salz der Zitronensäure mit einfach positiv geladenen Kationen und
b) mindestens einem Alkohol umfasst und
c) weder chaotrope Salze noch eine Kombination von Salzen mit mono- und multivalenten Kationen enthält und
d) dass die Ionenstärken für die Anbindung an die feste Phase in Kombination mit einem Alkohol kleiner als 100mM sind.

2. Verfahren nach Anspruch 1, **dadurch gekennzeichnet, dass** die Salze der Zitronensäure - Salze mit einfach positiv geladenen Kationen darstellen.

3. Verfahren nach Anspruch 2, **dadurch gekennzeichnet, dass** als Salze der Zitronensäure Hydrogencitrate oder Dihydrogencitrate eingesetzt werden.

4. Verfahren nach Anspruch 2 oder 3, **dadurch gekennzeichnet, dass** folgende Salze eingesetzt werden:
| | |
|---|---|
| a) Di-Ammoniumhydrogencitrat | und / oder |
| b) Ammoniumdihydrogencitrat | und / oder |
| c) Tri-Natriumcitrat | und / oder |
| d) Di-Natriumhydrogencitrat | und / oder |
| e) Natriumdihydrogencitrat | und / oder |
| f) Tri-Kaliumcitrat | und / oder |
| g) Di-Kaliumhydrogencitrat | und / oder |
| h) Kaliumdihydrogencitrat. | |

5. Verfahren nach einem der Ansprüche 1 bis 4, **dadurch gekennzeichnet, dass** die Alkoholkonzentrationen der Bindungspuffer zwischen 20% - 90% liegen.

6. Verfahren nach Anspruch 5, **dadurch gekennzeichnet, dass** die Alkoholkonzentrationen der Bindungspuffer zwischen 40% - 70% liegen.

7. Verfahren nach Anspruch 5 oder 6, **dadurch gekennzeichnet, dass** als Alkohole Methanol, Ethanol, Propanol, Isopropanol, Ethylenglycol, Polyethylenglycol oder Glycerin verwendet werden.

8. Verfahren nach einem der Ansprüche 1 bis 7, **dadurch gekennzeichnet, dass** die Ionenstärken für die Anbindung an die feste Phase in Kombination mit einem Alkohol kleiner als 50 mM sind.

9. Verfahren nach einem der Ansprüche 1 bis 8, **dadurch gekennzeichnet, dass** als feste Phase Glasfasermaterialien, Silicagele, Suspensionen aus mineralischen Trägern, funktionalisierte magnetische Partikel; vorzugsweise Glasfasermaterialien von 0.7 µm bis 2 µm verwendet werden.

10. Verfahren zur Trennung, Aufreinigung und Wiedergewinnung von lang- und / oder kurzkettigen Nukleinsäuren **gekennzeichnet durch** folgende Schritte:
➢ Zugabe der Bindungspuffer gemäß einem der Ansprüche 1 bis 8 zu Reaktionsansätzen, die Nukleinsäuren enthalten
➢ Überführung der Mischung aus den Reaktionsansätzen und den Bindungspuffern an eine feste Phase
➢ Elution der an die feste Phase gebundenen Nukleinsäuren.

11. Verfahren nach Anspruch 10, **dadurch gekennzeichnet, dass** ein Waschen der an die feste Phase gebundenen Nukleinsäuren entfällt.

12. Verwendung des Verfahrens gemäß den Ansprüche 1 bis 11 zur Aufreinigung von PCR-Produkten, Restriktionsansätzen oder Sequenzieransätzen.

## Claims

1. A method for the separation, purification and recovery of long- and / or short-chain nucleic acids with the following steps:
➢ bonding of the nucleic acids to a solid phase by means of a bonding buffer
➢ elution of the bonded nucleic acids from the solid phase,
**characterized in that** the bonding buffer
a) comprises as an active constituent a composition of at least one citric acid salt with cations having a single positive charge and
b) at least one alcohol and
c) neither does contain chaotropic salts nor a combination of salts with monovalent and multivalent cations and
d) that the ionic strengths for bonding to the solid phase in combination with an alcohol are smaller than 100mM.

2. A method according to claim 1, **characterized in that** the citric acid salts constitute salts with cations having a single positive charge.

3. A method according to claim 2, **characterized in that** hydrogen citrates or dihydrogen citrates are employed as citric acid salts.

4. A method according to claim 2 or 3, **characterized in that** the following salts are employed:
| | |
|---|---|
| a) Di-ammonium hydrogen citrate | and / or |
| b) ammonium dihydrogen citrate | and / or |
| c) trisodium citrate | and / or |
| d) Disodium hydrogen citrate | and / or |
| e) sodium dihydrogen citrate | and / or |
| f) tripotassium citrate | and / or |
| g) Di-potassium hydrogen citrate | and / or |
| h) potassium dihydrogen citrate. | |

5. A method according to any one of claims 1 to 4, **characterized in that** the alcohol concentrations of the bonding buffers are located between 20% - 90%.

6. A method according to claim 5, **characterized in that** the alcohol concentrations of the bonding buffers are located between 40% - 70%.

7. A method according to claim 5 or 6, **characterized in that** methanol, ethanol, propanol, isopropanol, ethylene glycol, polyethylene glycol or glycerin are used as alcohols.

8. A method according to any one of claims 1 to 7, **characterized in that** the ionic strengths for bonding to the solid phase in combination with an alcohol are smaller than 50 mM.

9. A method according to any one of claims 1 to 8, **characterized in that** as a solid phase glass fibre materials, silica gels, suspensions from mineral carriers, functionalized magnetic particles; preferably glass fibre materials from 0.7 µm to 2 µm are employed as a solid phase.

10. A method for the separation, purification and recovery of long- and / or short-chain nucleic acids **characterized by** the following steps:
➢ addition of the bonding buffer according to any one of claims 1 to 8 to reaction batches containing nucleic acids
➢ transfer of the mixture from the reaction batches and the bonding buffers to a solid phase
➢ elution of the nucleic acids bonded to the solid phase.

11. A method according to claim 10, **characterized in that** a washing of the nucleic acids bonded to the solid phase is omitted.

12. The employment of the method according to claim 1 to 11 for purification of PCR products, restriction batches or sequencing batches.

## Revendications

1. Procédé pour la séparation, la purification et la récupération des acides nucléiques à chaîne longue et / ou à chaîne courte avec les étapes suivantes:
➢ liaison des acides nucléiques à une phase solide au moyen d'un tampon de liaison
➢ élution des acides nucléiques liés de la phase solide, **caractérisé en ce que** le tampon de liaison,
a) comprend en tant que composant efficace une composition d'au moins un sel d'acide citrique portant une simple charge positive de cations et
b) au moins un alcool et
c) comprend ni des sels chaotropes ni une combinaison de sels avec des cations monovalents et multivalents et
d) que les forces ioniques pour la liaison à la phase solide en combinaison avec un alcool sont inférieures à 100mM.

2. Procédé selon la revendication 1, **caractérisé en ce que** les sels de l'acide citrique constituent des sels avec des cations portant une simple charge positive.

3. Procédé selon la revendication 2, **caractérisé en ce qu'**en tant que des sels de l'acide citrique des citrates d'hydrogène ou des citrates de dihydrogène sont utilisés.

4. Procédé selon la revendication 2 ou 3, **caractérisé en ce que** des sels suivants sont utilisés:
| | |
|---|---|
| a) citrate d'hydrogène de diammonium | et / ou |
| b) citrate de dihydrogène d'ammonium | et / ou |
| c) citrate de trisodium | et / ou |
| d) citrate d'hydrogène de disodium | et / ou |
| e) citrate de dihydrogène de sodium | et / ou |
| f) citrate de tripotassium | et / ou |
| g) citrate d'hydrogène de dipotassium | et / ou |
| h) citrate d'hydrogène de potassium. | |

5. Procédé selon l'une quelconque des revendications 1 à 4, **caractérisé en ce que** les concentrations en alcool des tampons de liaison sont comprises entre 20% - 90%.

6. Procédé selon la revendication 5, **caractérisé en ce que** les concentrations en alcool des tampons de liaison sont comprises entre 40% - 70%.

7. Procédé selon la revendication 5 ou 6, **caractérisé en ce qu'**en tant que des alcools sont utilisés du méthanol, d'éthanol, du propanol, d'isopropanol, d'éthylène glycol, du polyéthylène glycol ou de la glycérine

8. Procédé selon l'une quelconque des revendications 1 à 7, **caractérisé en ce que** les forces ioniques pour la liaison à la phase solide en combinaison avec un alcool sont inférieures à 50 mM.

9. Procédé selon l'une quelconque des revendications 1 à 8, **caractérisé en ce qu'**en tant que phase solide sont utilisés des matériaux en fibre de verre, des gels de silice, des suspensions à partir de porteurs minéraux, des particules magnétiques fonctionnalisées; de préférence des matériaux en fibre de verre de 0,7 µm à 2 µm.

10. Procédé pour la séparation, la purification et la récupération des acides nucléiques à chaîne longue et / ou à chaîne courte **caractérisé par** les étapes suivantes:
➢ addition des tampons de liaison selon l'une des revendications 1 à 8 aux mélanges réactionnels contenant des acides nucléiques
➢ transfert du mélange à partir des mélanges réactionnels et des tampons de liaison à une phase solide
➢ élution des acides nucléiques liés à la phase solide.

11. Procédé selon la revendication 10, **caractérisé en ce qu'**un lavage des acides nucléiques liés à la phase solide est supprimé.

12. Utilisation du procédé selon les revendications 1 à 11 pour la purification des produits PCR, des mélanges de restriction ou de séquençage.
